# EUROPEAN PATENT APPLICATION

(11) **EP 0 791 365 A2**
(43) Date of publication of application: **27.08.1997**
(21) Application number: 96119306.7
(22) Date of filing: 02.12.1996
(51) Int. Cl.: A61L 9/12

(54) **Air cleaner**

(30) Priority: 26.02.1996 JP 38470/96
(71) Applicant: Cosmo Ace Co., Hiroshima-shi, Hiroshima 731-01 (JP)
(72) Inventor: Nishimoto, Teturou, Cosmo Ace Co., Hiroshima-shi, Hiroshima 731-01 (JP); Nishimoto, Kiyohiro, Cosmo Ace Co., Hiroshima-shi, Hiroshima 731-01 (JP)
(74) Representative: TER MEER STEINMEISTER & PARTNER GbR

(57) **Abstract**

To provide an air cleaner in which required capacity is obtained even through a small fan, so that cost, space, noise and the like of the air cleaner are effectively reduced. In the air cleaner, a fan 15 for inducing air to a main body is mounted; chemical 20 is stored in the main body; a filter 21 through which the chemical percolates by capillary effect is mounted; air induced in the main body by the fan 15 is extracted to outside after being in contact with the chemical 20 and the filter 21; and the filter 21 is steeped in the chemical 20 at a portion thereof, and is arranged along a stream of the air which is induced to the main body and extracted to outside by the fan 15.

## Description

### BACKGROUND OF THE INVENTION

### 1. Field of the Invention

The present invention relates to an air cleaner for cleaning air in rooms in a medical facility, a greenhouse, a henhouse, a food manufacturing plant or a general home, for instance, and more particularly to an air cleaner in which air is induced and contacts with chemical with antibacterial effect and the like and a filter while passing through the inside of the air cleaner, so that not only mold, virus, tick, dust, pollen and the like are killed and removed, but also toxic gas such as ethylene and carbon dioxide are removed.

### 2. Description of the Related Art

In recent years, an air cleaner for killing and removing substances suspending in air and harmful to human being, animals and plants (hereinafter referred to as "bacilli and the like" such as mold, virus, tick, dust, pollen and minute particles as an origin of bad smell, which cannot be cleaned or trapped by an air cleaner for removing ordinary dusts or smoke of cigarette (thorough ordinary filter, active carbon, ion or the like) has been eagerly desired.

Especially, in recent years, so-called hospital infection of MRSA or the like has become a problem. In order to prevent such hospital infection, it has been earnestly desired that an air cleaner capable of killing and removing bacilli and the like in rooms in a ward, a consultation room, a waiting room or the like which is in danger of contamination. Such air cleaner has already been prepared or used in several facilities.

In agricultural and stockbreeding fields also, for instance, it has been trying to mount an air cleaner with antibacterial effect to reduce bacterium causing gray mold or powdery mildew in a greenhouse, bacilli causing Newcastle disease and the like through airborne infection in a henhouse or bacilli such as Staphylococcus or salmonella germ causing food poisoning. Further, in general home also, it is desired to mount such air cleaner for the purpose of preventing atopic allergy, improving physical condition, promoting health, and mental stabilization.

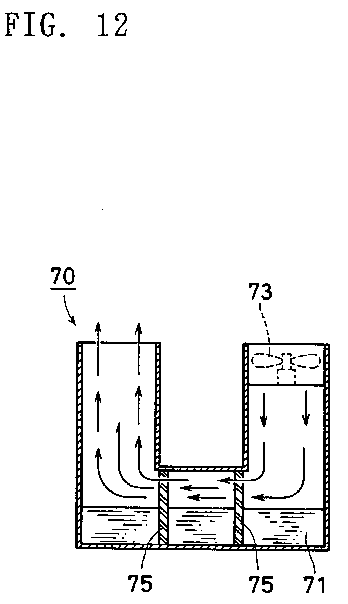

A conventional air cleaner with antibacterial effect is provided with a fan permitting air induced in a body of the air cleaner to be in contact with chemicals (such as antibacterial liquid), which is schematically illustrated in Fig. 12 by the applicant of the present invention. In the air cleaner 70, chemical 72 is stored at a bottom portion thereof, and air induced by a fan 73 is extracted to outside with the air being in contact with the surface of the chemical 71. Two filters 75 are disposed in parallel with each other, and the lower portions thereof are steeped into the chemical 71 to introduce the chemical 71 to the upper portion of the filters through capillary effect. The stream of the air containing bacilli and the like passes through the upper portion of the filter 75 while being in contact with the chemical 71 to kill and remove the bacilli and the like.

It is required for the air cleaner with antibacterial effect described above that bacilli and the like suspending in the air in rooms is readily killed and removed to effectively restrict the increase of the bacilli. Therefore, it is also necessary that the air cleaner have the capacity for allowing the air in the rooms to circulate in a predetermined period of time. Although the air induced in the body of the air cleaner is being in contact with chemical to obtain a certain antibacterial effect in the conventional air cleaner described above, since the filter is disposed so as to counteract the stream of the air, in other words, the air passes the filter in a direction of the thickness thereof, air resistance becomes remarkably strong, so that it is indispensable to install a fan with considerably large capacity in comparison to the dimension of the room to be cleaned to provide the above-mentioned capacity.

However, in general, not only a fan is becomes larger and more expensive as the capacity thereof becomes larger, but also noise and power consumption also increases, causing problems of cost, space and noise protection.

The present invention has been made in consideration of the problems described above, and the object thereof is to provide an air cleaner with a small fan capable of providing a required capacity to effectively reduce the cost, space and noise.

### SUMMARY OF THE INVENTION

In order to accomplish the object described above, basically, in an air cleaner according to the present invention, a fan is mounted to induce air to a main body; chemical is stored in the main body; a filter, to which the chemical is introduced through capillary effect, is mounted; and the air induced into the main body by the fan contacts the chemical and the filter, and then extracted out of the main body.

The filter is steeped in the chemical at a part thereof and is arranged along the stream of the air induced in and extracted from the main body by the fan.

As the filter described above, any filter with capillary effect, such as unwoven cloth and water suction paper, for introducing chemical may be selected.

In the air cleaner according to the present invention, the filter is arranged, wherever practicable, so as not to block the stream of the air which is induced and extracted from the main body by the fan. Concretely, the filter is arranged in such a manner as to be in parallel to or slightly inclined to the stream of the air, and the air does not pass through the filter but it flows along the outer surface of the filter while contacting it. More preferably, a plurality of filters may be arranged in parallel to the stream of the air and they perpendicularly cross with each other, or a filter may be arranged spirally along the stream of the air.

In the air cleaner according to the present invention with the above-mentioned construction, the air induced by the fan flows while contacting with the chemical and outer surfaces of the filters containing the chemical which extends in a longitudinal direction thereof not in a direction of its thickness. Therefore, air resistance of the filters can be reduced remarkably in comparison with conventional one, which provides required capacity even through a small fan, resulting in effective reduction in cost, space and noise.

In this case, bacilli and the like suspending in air is killed when contacting the chemical in the tank or the chemical percolating the filters, and they are trapped there, so that the trapped bacilli and the like can not be returned to the air again. This may be explained as the same reason that dust does not suspend in air after watering at cleaning, or air is clean without dust after raining.

In addition to the above, the chemical in the tank and the chemical percolating the filters evaporate in accordance with the humidity (dryness) of the air which flows while being in contact with the chemical in the tank and filters in the main body, which causes the humidity in the room to be adjusted naturally and the mist of the chemical to be released in the air. The air containing the evaporated mist of the chemical is blown from the main body so as to be returned in the room, preventing the increase of mold, virus or the like. Further, the air is directly induced into human body through breathing and skin, contributing improvement of physical condition. The chemical percolates the filter through capillary effect, so that the amount of evaporated chemical may be adjusted by shape and dimension, arrangement, and material of the filter, which also controls the humidity properly.

The air cleaner according to the present invention is used in rooms in a medical facility, a greenhouse, a henhouse, a food manufacturing plant or a general home, for instance, however, the use of the air cleaner is not limited to these facilities. The chemical (with antibacterial effect or the like) may properly be selected in accordance with the use and the place that the air cleaner is installed, that is, the kind of bacilli or the like which should be killed and removed.

As the chemical, for example, hinokitiol solution is preferable for general home use. The hinokitiol is crystallized acid material contained in Taiwan cypress oil, Aomori Thujopsis dolabrate oil and the like. Antibacterial effect of the hinokitiol prevent the increase of tick, and improves immunocompetence of human body, and is effective against allergy and atopy as well. The hinokitiol does not cause bacilli and virus to have resistance, and does not harmful to human body, and is effective to hospital infection of MRSA or the like. Further, the hinokitiol has osmotic action so as to be absorbed through breathing and skin, which contributes the improvement of physical condition of a person with atopic allergy. Besides, the hinokitiol has aroma effect.

In case that decoction of eye drops tree known as Chines medicine as chemical for medical use and for general home, it is expected to have an effect to eye strain or the like.

As chemical for agricultural use which is used in a greenhouse or the like, activated silicone liquid and multi-element mineral liquid are used, which are effective against germ causing bine dead disease, gray mold, powdery mildew and the like. Further, as chemical for use in food manufacturing plant and henhouse, stabilized chlorine dioxide liquid is used since it is effective against bacilli causing Newcastle disease and the like through airborne infection in a henhouse or bacilli such as Staphylococcus or salmonella germ causing food poisoning.

### BRIEF DESCRIPTION OF THE DRAWINGS

The present invention will be more apparent from the ensuring description with reference to the accompanying drawings wherein:
Figure 1 is a lateral cross-sectional perspective view of an air cleaner according to the first embodiment of the present invention;
Figure 2 is a longitudinal cross-sectional view of the air cleaner illustrated in Fig.1;
Figure 3 is a lateral cross-sectional view for explaining the effect of the air cleaner shown in Fig.1;
Figure 4 is a longitudinal cross-sectional perspective view of the bottom portion of an air cleaner according to the second embodiment of the present invention;
Figure 5 is a lateral cross-sectional perspective view of an air cleaner according to the third embodiment of the present invention;

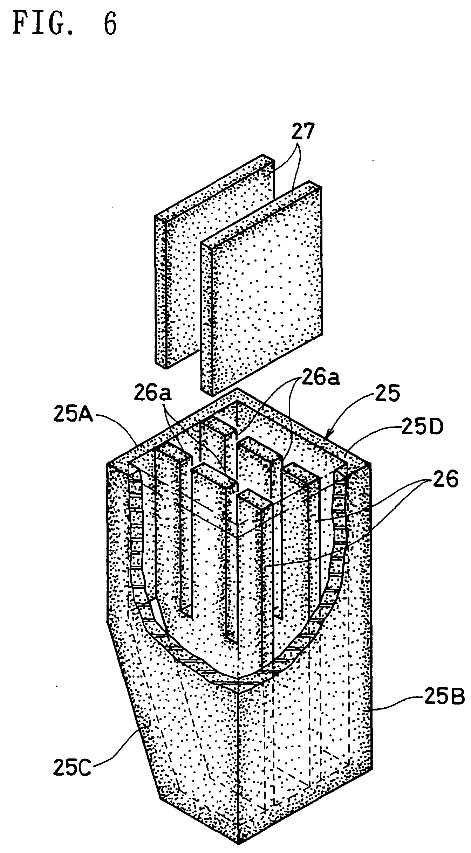

Figure 6 is a fragmented perspective view of the filter for use in the air cleaner shown in Fig. 5;
Figure 7 is a lateral cross-sectional view of an air cleaner according to the fourth embodiment of the present invention;
Figure 8 is a partial perspective view of an air sampler which is used for confirming the effect of the air cleaned according to the present invention;
Figure 9 shows the conditions of waiting room (a), consultation room (b), and operating room (c) at A hospital before (up) and after (down) using agar strip;
Figure 10 shows the conditions of waiting room (a), consultation room (b), and operating room (c) at B hospital before (up) and after (down) using agar strip;
Figure 11 shows the conditions of waiting room (a), consultation room (b), and operating room (c) at C hospital before (up) and after (down) using agar strip; and
Figure 12 is a lateral cross-sectional view of a conventional air cleaner.

### DETAILED DESCRIPTION OF PREFERRED EMBODIMENTS

Now, an air cleaners according to embodiments of the present invention will be described with reference to drawings.

### [first embodiment]

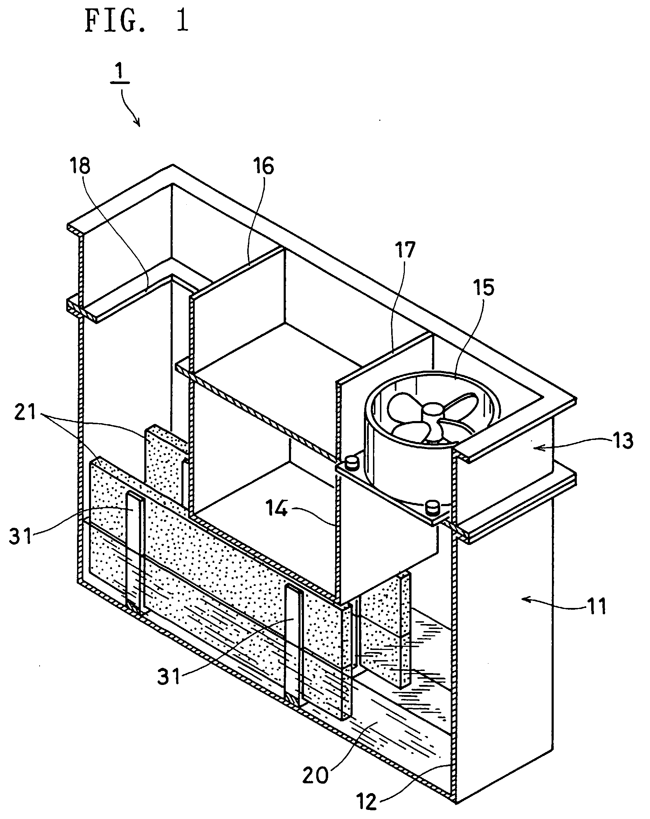

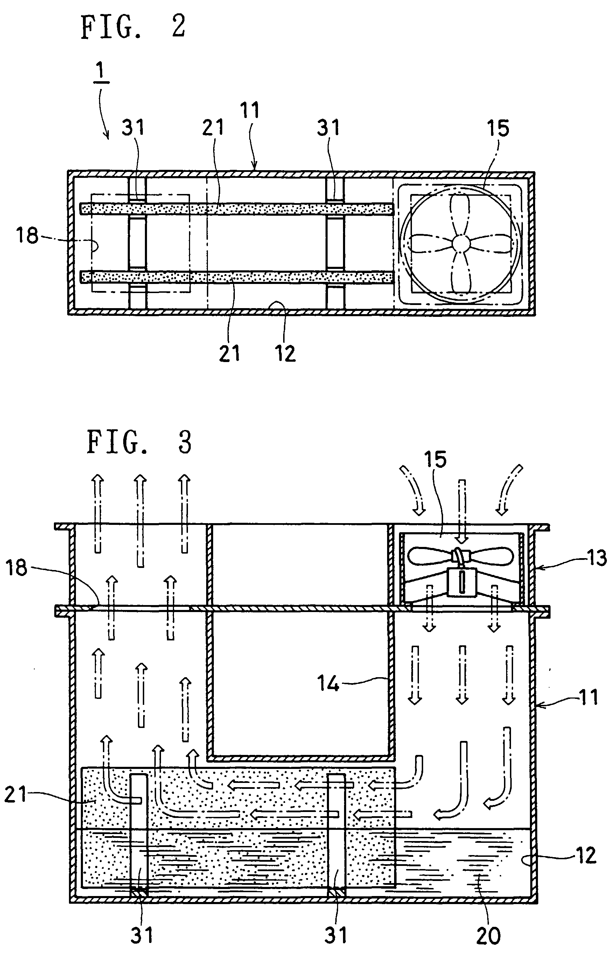

Figure 1 schematically shows an air cleaner according to the first embodiment of the present invention, and Fig. 2 is a horizontal cross-sectional view of this air cleaner. The air cleaner 1 in the figures includes a box-shaped main body made of steal plate or synthetic resin, and an upper case 13 in which a fan 15 is situated at a right side thereof. A bottom portion of the main body 11 is used as a tank 12 for chemical 20 as described below. In the middle of the main body is disposed a U-shaped passage forming plate 14. On the right and left sides of the central portion of the upper case 13 are located partition plates 16, 17, and to the left of the partition plate 16 is formed an air outlet 18.

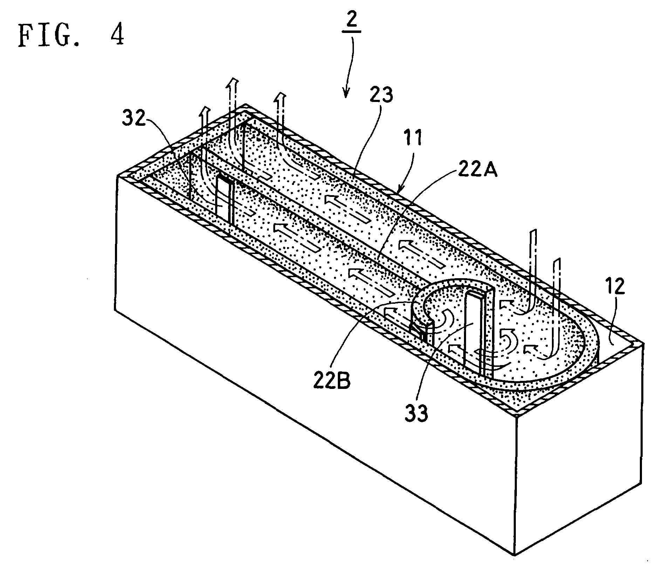

In the air cleaner 1, air induced by the fan 15 passes through a passage which is formed between the main body 11 and the passage forming plate 14 over the surface of the chemical 20 from right to left in such a manner as to write the character U in reverse direction and the air is finally extracted from air outlet 18 to outside, that is, into rooms, as indicated by phantom arrow marks in Fig. 3.

In the air clear according to the present invention, two pieces of rectangular plate-shaped filters 21 with the same dimensions are separately and vertically supported by supporting members 31, and the lower portions of the filters 21 are steeped in the chemical 20. Prescribed spaces are provided between ends of the filters and front and rear inner walls of the main body 11, and the filters 11 are disposed so as to oppose with each other at a predetermined distance in between and are arranged in the same direction as the stream of the air (horizontal direction in this embodiment) which is induced into the main body 11 and extracted to outside by the fan 15.

These filters 21 are made of unwoven sheet, and the chemical 20 goes up to the upper portion of the filters through capillary effect. Concretely, for instance, a bottom-steeped mat "love mat U", which is a registered trademark, from Unitika Ltd. can be used as a filter for the above purpose.

A dust filter and ducts for adjusting the directions of the induced and extracted air (not shown in the figures) may be attached to and installed on the fan 15 of the upper case 13 and air outlet 18 respectively, as occasion demands. The main body 11 is provided with openings and the like for renewing the chemical 20 and the filters 21 as well as for inspection.

With the construction of the air cleaner 1 according to this embodiment of the present invention, the air induced in the main body 11 by the fan 15 flows while contacting with the chemical 20 and outer surfaces of the filters 21 containing the chemical 20 which extends in a longitudinal direction thereof not in a direction of its thickness. Therefore, air resistance of the filters 21 can be reduced remarkably in comparison with a conventional air filter in which filters are disposed so as to counteract the stream of air, which allows required capacity even through a small fan, resulting in effective reduction in cost, space and noise.

In the above embodiment, bacilli and the like suspending in air is killed when contacting the chemical 20 in the tank 12 or the chemical 20 percolating the filters 21 and trapped there, so that they can not be returned to the air again.

In addition to the above, the chemical 20 in the tank 12 and the chemical 20 percolating the filters 21 evaporate in accordance with the humidity of the air which flows while being in contact with the chemical 20 in the tank 12 and filters 21 in the main body 11, which causes the humidity in the room to be adjusted naturally and the mist of the chemical to be released in the air. The air containing the evaporated mist of the chemical is blown from the main body 11 so as to be returned in the room, preventing the increase of mold, virus or the like. Further, the chemical 20 percolates the filters 21 through capillary effect, so that the amount of evaporated chemical may be adjusted with dimension, shape, arrangement and material thereof, which also allows the humidity to be adjusted properly.

The chemical 20 (with antibacterial effect or the like) for use in the above-mentioned air cleaner 1 may properly be selected in accordance with the use and place that the air cleaner 1 is installed, that is, the kind of bacilli or the like which should be killed and removed. The use and representative composition (weight %) of the chemical is shown below.
(1) Chemical for agricultural use
   (a) Activated silicone liquid
      - Silicone:: 0.004 %
      - Calcium:: 0.91 %
      - Magnesium:: 0.0072 %
      - Water:: remainder
   (b) Multi-element mineral liquid
      - enzyme:: 0.5 %
      - Mineral:: 0.8 %
      - humin:: 7.3 %
      - Microorganisms:: trace
      - Water:: appro. 91 %
(2) Chemical for food manufacturing plant and henhouse
   Stabilized chlorine dioxide liquid
      - ClO₂ (effective component):: 6 %
      - Na₂CO₃:: 1%
      - Na₂SO₄:: trace
      - Water:: appro. 93 %
(3) Chemical for medical use and for general home
   - Hinokitiol:: 0.03 %
   - Water:: remainder

### [Example of practical test]

### (A) Test method

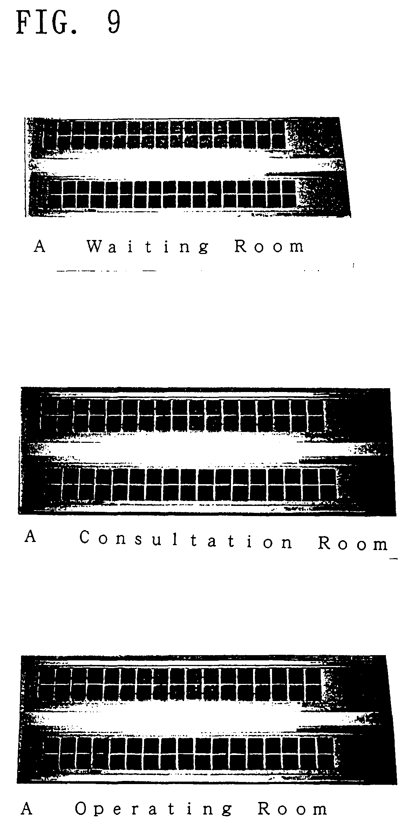

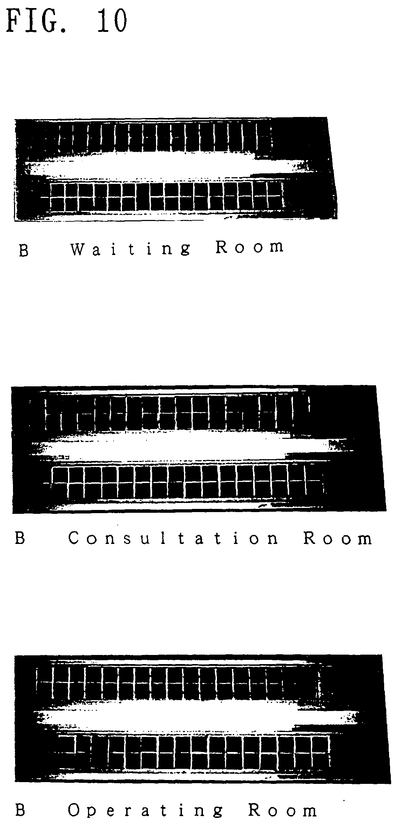

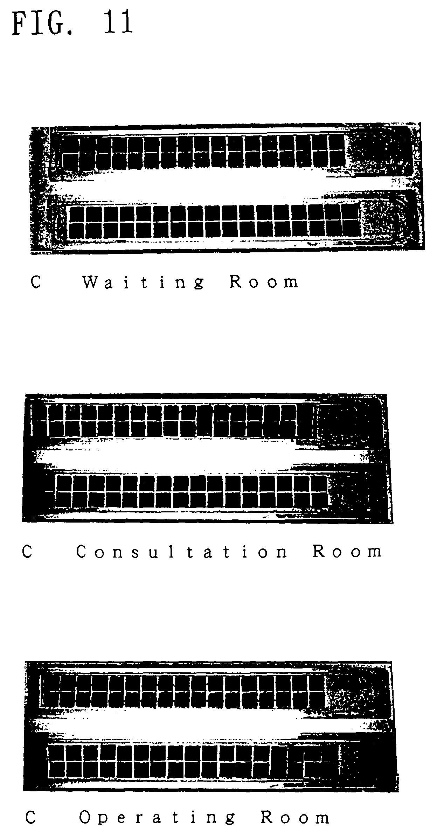

In order to confirm antibacterial effect of the air cleaner 1 with the aforementioned construction, the air cleaner 1 is installed and is operated for 12 hours under the same conditions (described below) in waiting room, consultation room, and operating room at A, B and C hospitals. After the operation of the air cleaner 1, a predetermined quantity of air is sampled from each room by a sampling device described below and the number of bacilli contained in the air is counted. The test results are shown in tables 1, 2 and 3. Figures 9, 10 and 11 illustrate a medium (agar strip) for use in the sampling device in the waiting room, consultation room, and operating room at A, B and C hospitals before and after operation.

### (B) Test conditions

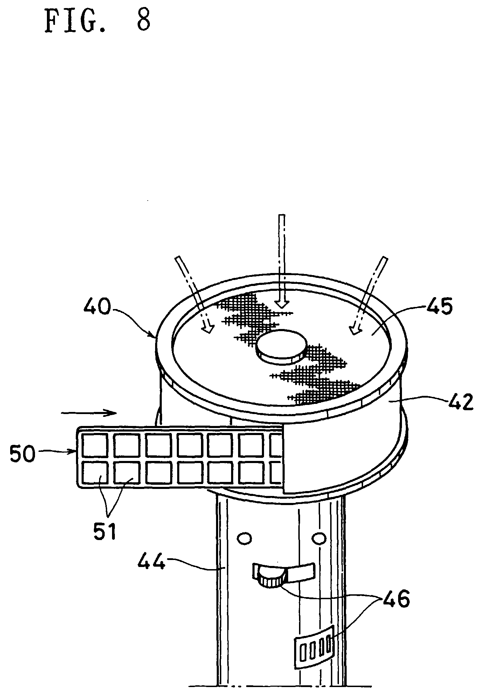

(1) A multiblade fan is used for the air cleaner. The specification of the fan is: power source is 100V; amperage 0.23A; air volume 142 m³/h (60Hz); and statistic pressure 200 pa (at air volume of 0 m³/h).
(2) The air cleaner is operated for 12 hours.
(3) The temperature and humidity in the air is approximately 20 C and 30% respectively.
(4) One circulation (one revolution) of the air in a room (hour/rev. in table 1) is calculated by dividing the volume of the room by air volume of the fan (142m³/h).
(5) The sapling device used is an air sampler 40 from Biotest Ltd. in Germany, as illustrated in Fig. 8. In the air sampler 40, exclusive medium (agar strip) with a prescribed number of sections 51 is attached to the inner periphery of a cylindrical portion 42 at the top of the main body 44 with operation dials, switches 46 and the like, and a predetermined amount of air is induced by a rotating blades accommodated in the cylindrical portion 42 with a mesh filter 45. The medium used is for general bacilli and they are cultivated for three days at 20 C.

### (C) Test results

As clearly understood from Tables 1 to 3 and Figs. 9 to 11, with the air cleaner 1, The number of bacilli and the like suspending in the air are drastically reduced.

**[Table 1]**

| A hospital | | | |
|---|---|---|---|
| | Number of Bacilli | | |
| | Waiting Room | Consultation Room | Operating Room |
| Before Operation | 517 | 253 | 236 |
| After Operation | 18 | 5 | 11 |
| Reduction Ratio | 1/28 | 1/50 | 1/21 |
| Volume (m³) | 930 | 430 | 875 |
| Time (hour/rev.) | 6.5 | 3.0 | 6.1 |

**[Table 2]**

| B hospital | | | |
|---|---|---|---|
| | Number of Bacilli | | |
| | Waiting Room | Consultation Room | Operating Room |
| Before Operation | 633 | 281 | 209 |
| After Operation | 21 | 5 | 10 |
| Reduction Ratio | 1/30 | 1/56 | 1/20 |
| Volume (m³) | 775 | 150 | 850 |
| Time (hour/rev.) | 5.4 | 1.0 | 5.9 |

**[Table 3]**

| C hospital | | | |
|---|---|---|---|
| | Number of Bacilli | | |
| | Waiting Room | Consultation Room | Operating Room |
| Before Operation | 411 | 278 | 213 |
| After Operation | 17 | 6 | 9 |
| Reduction Ratio | 1/26 | 1/46 | 1/23 |
| Volume (m³) | 1075 | 500 | 470 |
| Time (hour/rev.) | 7.5 | 3.5 | 3.3 |

### [Second embodiment]

Figure 4 schematically illustrates the bottom portion of an air cleaner 2 according to the second embodiment of the present invention. The air cleaner 2 differs from the air cleaner 1 in the shape and the arrangement of filters, however, other parts and elements of the air filters 1 and 2 are the same. In the air cleaner 2, a filter 23 with a round portion at an one end is disposed along the inner face of the tank 12 of the main body 11, and a plate-shaped filter 22A is located from the left end of the tank 12 to slightly right through the center of the tank 12. Further, a gutter-shaped filter 22B is positioned with an opened portion thereof directing right.

The above-mentioned filters 23, 22A and 22B are made of unwoven cloth like the filters 21, 22 in the first embodiment shown in Fig 1, and the chemical 20 goes up to the upper portions of the filters 23, 22A, 22B through capillary effect. The filters 23, 22A, 22B are vertically stand so as to be steeped in the chemical 20 at the lower portions thereof and are arranged along the stream of the air, which is induced in the main body 11 and extracted to outside by the fan 15. The filters 22A and 22B are supported by supporting members 32, 33 so as not to fall down.

In the air cleaner 2 with the aforementioned construction also, the air induced to the main body 11 by the fan 15 flows while contacting with the chemical 20 and outer surfaces of the filters 23, 22A, 22B containing the chemical 20 which extends in a longitudinal direction thereof not in a direction of its thickness. In this case, although the air strikes the filter 22B, since there is a space between the filters 22B and 23, the air is not blocked by the filter 22B so strongly and flows while being in contact with outer surfaces of the filters 23, 22A, and 22B as indicated by arrows with phantom lines. As a result, the air cleaner 2 in this embodiment has the same effect as the first embodiment, and the contact area between the air and the filters is increased in comparison to that of the first embodiment, resulting in improved antibacterial effect.

### [Third embodiment]

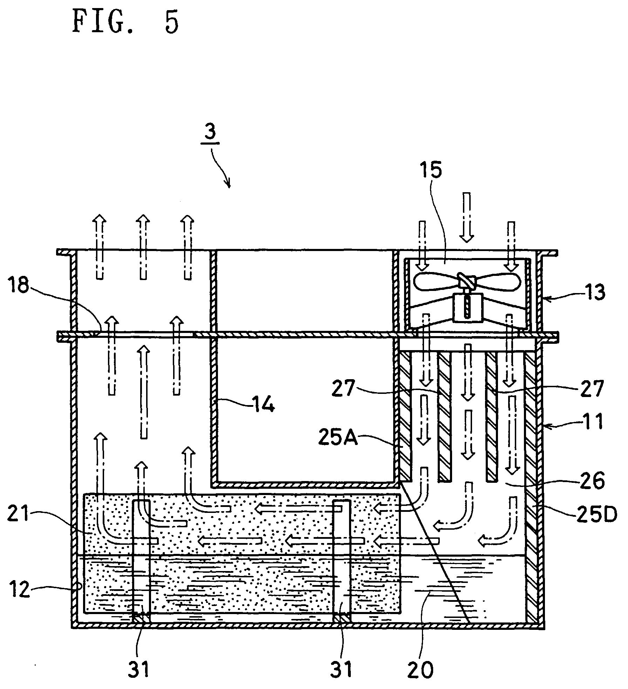

Figure 5 is a schematic view of an air cleaner 3 according to the third embodiment of the present invention. The air cleaner 3 also differs from the filters 1, 2 in shape and arrangement of filters, however, material and other parts of the air filters are the same. In the air cleaner 3, filters 21 are mounted at the tank 12 in the same manner as the first embodiment in Fig. 1, and on the right side of the filters, that is, below the fan 15 are positioned filters 25, 26, 27, which are assembled to form lattice as illustrated in Fig. 3. The filters 25, 26, 27 are arranged along the stream of the air (vertically in this embodiment) induced in the main body 11 and extracted to outside by the fan 15.

The filter 25 of these filters, which is situated outer side among the filters 25, 26, 27, consists of four plate filter elements 25A, 25B, 25C, 25D. The plate filter element 25A is relatively short, and is attached to the right side wall of the passage forming plate 14, and does not extend toward the tank 12 below. The plate filter element 25B is attached to the inner right side wall, and the lower portion of the filter element 25B is steeped in the chemical 20 in the tank 12. The plate filter elements 25C and 25D have the same length as the filter element 25B, and the lower portions thereof are steeped in the chemical 20 in the tank 12.1 The left side of the filter elements 25C and 25D are partially cut. Two filters 26, which are located inside of the rectangular filters 25 (shown from upside), have the same shapes as those of the filters 25C and 25D. Notches 26a are formed on the upper portion of each filter 26 to mount two rectangular-plate-shaped filters 27. Like the plate filter 25, the filters 27 are relatively short and do not project toward the tank 12.

In the air cleaner 3 with the aforementioned construction also, the air induced to the main body 11 by the fan 15 flows while contacting with the chemical 20 and outer surfaces of the filters 25, 26, 27, 21 containing the chemical 20 which extends in a longitudinal direction thereof, that is, in a direction shown by the arrows with phantom lines, not in a direction of its thickness. As a result, the air cleaner 3 in this embodiment has the same effect as the first and second embodiments, and the contact area between the air and the filters is increased in comparison with those of the first and second embodiments, resulting in improved antibacterial effect.

### [Fourth embodiment]

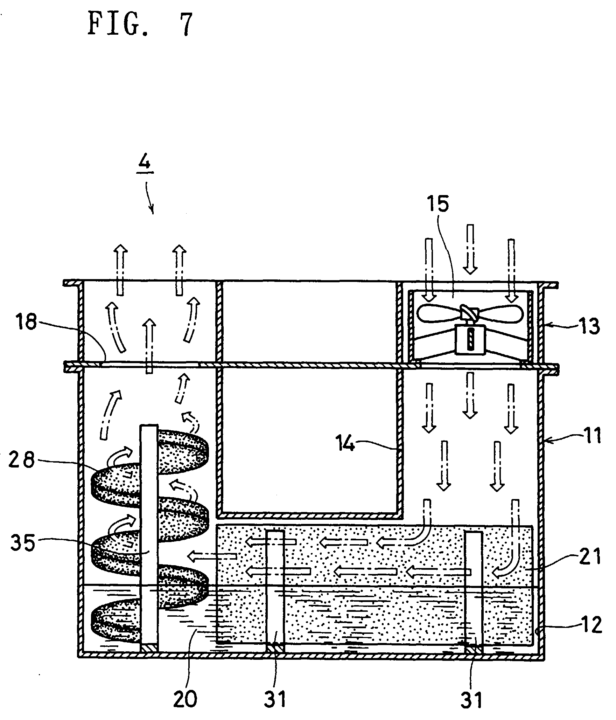

Figure 7 is a schematic view of an air cleaner 4 according to the fourth embodiment of the present invention. The air cleaner 4 also differs from the filters 1, 2, 3 in shape and arrangement of filters, however, material and other parts of these air filters are the same. In the air cleaner 4, filters 21 are mounted at the tank 12 on right side in the same manner as the first embodiment in Fig. 1, and on the left side of the filters 21, that is, below the air outlet 18 is positioned a filter 28 consisting of tab and semicircle filter elements, which are connected to each other. The filter 28 is supported by a pair of supporting members (only one member is illustrated) at the front and rear peripheral portions thereof. The filter 28 is arranged to form a spiral along the stream of the air (vertically in this embodiment) induced in the main body 11 and extracted to outside by the fan 15. The lower portion of the filter 28 is steeped in the chemical 20 in the tank 12, and the chemical 20 percolates the filter 28 from the lower portion to the upper portion of the filter 28.

In the air cleaner 4 with the aforementioned construction also, the air induced to the main body 11 by the fan 15 spirally flows while contacting with the chemical 20 on upper and lower surfaces of the filters 21, 28 containing the chemical 20, that is, in a direction shown by the arrows with phantom lines in Fig. 7, not in a direction of its thickness. As a result, the air cleaner 4 in this embodiment has the same effect as the first and second embodiments, and the contact area between the air and the filters is increased in comparison with those of the first and second embodiments, resulting in improved antibacterial effect.

As understood from the above explanation, with air cleaners according to the present invention, the air resistance of filters are remarkably reduced in comparison with conventional one. Therefore, required capacity is obtained even with a small fan, and cost relating the air cleaner, space for the leaner and noise generated by the cleaner are effectively reduced.

Upon contacting with chemical and filters, bacilli and the like suspending in air are decreased in numbers since they are killed and trapped therein. The chemical that stored in tank and percolates the filters evaporates in accordance with the humidity of the air which flows while contacting the chemical in the tank and the filters in the main body, which causes the humidity in the room to be naturally adjusted and the chemical mist to be released in the air, preventing the increase of the mold, virus and the like in the room.

## Claims

1. An air cleaner in which a fan (15) for inducing air in a main body is mounted; chemical (20) is stored in the main body (11); a filter (21) through which the chemical (20) percolates by capillary effect is mounted; and the air induced in the main body (11) by the fan (15) is extracted to outside after being in contact with the chemical (20) and the filter (21) **characterized in that** said filter (21) is steeped in said chemical (20) at a portion thereof, and is arranged along a stream of the air which is induced to the main body (11) and extracted to outside by the fan.

2. The air cleaner as claimed in claim 1, wherein said filter is located in parallel with said stream of air which is induced in the main body and extracted to outside by the fan.

3. The air cleaner as claimed in claim 1, wherein a plurality of said filters are arranged in parallel with said stream of air which is induced in the main body and extracted to outside by the fan, and said filters cross perpendicularly with each other.

4. The air cleaner as claimed in claim 1, wherein said filter is spirally mounted to said main body along the stream of air which is induced in the main body and extracted to outside by the fan.

5. The air cleaner as claimed in one of claims 1 to 4, wherein said chemical is one of hinokitiol solution, activated silicone liquid, multi-element mineral liquid, and stabilized chlorine dioxide liquid.

6. A method of cleaning air using said air cleaner claimed in claim 1 and hinokitiol solution as said chemical.
